# EUROPÄISCHE PATENTSCHRIFT

(11) **EP 1 676 828 B1**
(45) Veröffentlichungstag und Bekanntmachung des Hinweises auf die Patenterteilung: **05.08.2009**
(21) Anmeldenummer: 05110309.1
(22) Anmeldetag: 03.11.2005
(51) Int. Cl.: C07C 67/303, C07C 69/75, C08K 5/12

(54) **Verfahren zur Herstellung von alicyclischen Carbonsäuren oder deren Derivaten**
Process for the preparation of alicyclic carboxylic acids or derivatives thereof
Procédé de préparation d'acides carboxyliques alicycliques ou de leur derivés

(30) Priorität: 31.12.2004 DE 102004063637
(43) Veröffentlichungstag der Anmeldung: 05.07.2006
(73) Patentinhaber: Evonik Oxeno GmbH, 45772 Marl (DE)
(72) Erfinder: Graß, Michael, 45721, Haltern am See (DE); Reeken, Burkhard, 46282, Dorsten (DE); Tuchlenski, Axel, 69469, Weinheim (DE); Kaizik, Alfred, 45772, Marl (DE); Büschken, Wilfried, 45721, Haltern am See (DE)
(74) Vertreter: Hirsch, Hans-Ludwig

(56) Entgegenhaltungen:
- WO-A-94/29261
- DE-A1- 10 225 565
- DE-A1- 10 232 868

## Beschreibung

Die Erfindung betrifft die Herstellung von alicyclischen Carbonsäureestern durch Selektivhydrierung der entsprechenden aromatischen Carbonsäure(-derivate)n in mindestens drei hintereinandergeschalteten Reaktoren, wobei mindestens die beiden ersten in Schlaufenfahrweise betrieben werden.

Alicyclische Polycarbonsäureester, wie beispielsweise die Ester der Cyclohexan-1,2-dicarbonsäure, werden als Schmierölkomponente und als Hilfsmittel bei der Metallverarbeitung eingesetzt. Weiterhin finden sie als Weichmacher für Polyolefine und für PVC Verwendung.

Für die Weichmachung von PVC werden überwiegend Ester der Phthalsäure, wie beispielweise Dibutyl-, Dioctyl-, Dinonyl- oder Didecylester der Phthalsäure, verwendet. Da über die Verwendung dieser Phthalate in letzter Zeit zunehmend kontrovers diskutiert wird, könnte deren Einsatz in Kunststoffen eingeschränkt werden. Alicyclische Polycarbonsäureester, von denen einige in der Literatur bereits als Weichmacher für Kunststoffe beschrieben sind, könnten dann als geeignete Ersatzstoffe Verwendung finden.

In den meisten Fällen ist der wirtschaftlichste Weg zur Herstellung von alicyclischen Polycarbonsäureestern die Kernhydrierung der entsprechenden aromatischen Polycarbonsäureester, beispielsweise der o. g. Phthalate. Es sind hierzu bereits einige Verfahren bekannt:

In US 5,286,898 und US 5,319,129 werden Verfahren beschrieben, mit denen Dimethylterephthalat an geträgerten Pd-Katalysatoren, die mit Ni, Pt und/oder Ru dotiert sind, bei Temperaturen größer oder gleich 140 °C und einem Druck zwischen 50 und 170 bar zum entsprechenden Hexahydrodimethylterephthalat hydriert werden kann.

US 3,027,398 offenbart die Hydrierung von Dimethylterephthalat an geträgerten Ru-Katalysatoren bei 110 bis 140 °C und 35 bis 105 bar.

In DE 28 23 165 werden aromatische Carbonsäureester an geträgerten Ni, Ru, Rh und/oder Pd-Katalysatoren zu den entsprechenden alicyclischen Carbonsäureestern bei 70 bis 250 °C und 30 bis 200 bar hydriert. Dabei wird ein makroporöser Träger mit einer mittleren Porengröße von 70 nm und einer BET-Oberfläche von ca. 30 m²/g eingesetzt.

Weitere Ruthenium-Trägerkatalysatoren für die Herstellung von alicyclischen Polycarbonsäureestern durch Hydrierung von aromatischen Polycarbonsäureestern werden in den Schutzrechtsdokumenten WO 99/32427, WO 00/78704, DE 102 25 565.2 und DE 102 32 868.4 beansprucht.

In WO 2004/046078 wird die Hydrierung von Benzolpolycarbonsäuren oder deren Derivaten an einem Katalysator beschrieben, der das aktive Katalysatormetall aufgebracht auf einem Träger aufweist, wobei der Träger ein oder mehrere Materialien mit geordneten Mesoporen aufweist.

Die Hydrierung der aromatischen Polycarbonsäureester erfolgt in US 3,027,398 ansatzweise, in US 5,286,898, US 5,319,129, DE 28 23 165, WO 99/32427 und WO 00/78704 kontinuierlich in einem Rohrreaktor ohne oder mit Rückführung (Schlaufenfahrweise) des Hydrieraustrags.

In DE 102 32 868.4 und DE 102 25 565.2 wird die Hydrierung der aromatischen Polycarbonsäureester zu den entsprechenden alicyclischen Polycarbonsäureestern in zwei hintereinandergeschalteten Reaktoren durchgeführt, wobei der erste in Schlaufenfahrweise (teilweise Rückführung des Reaktoraustrages) und der zweite im geraden Durchgang betrieben wird. Der erste Schlaufenreaktor kann auch durch mehrere kleine in Reihe oder parallel verschaltete Schlaufenreaktoren ersetzt werden, wobei diese Reaktoren einen gemeinsamen Kreislauf aufweisen.

Da die technisch bekannten Verfahren hinsichtlich der Raum-Zeit-Ausbeute und/oder der Selektivität nicht voll befriedigen, bestand die Aufgabe, ein Hydrierverfahren zu entwickeln, das einen oder mehrere dieser Nachteile nicht aufweist und ein Hydrierprodukt liefert, das als Es wurde nun gefunden, dass bei der Hydrierung von aromatischen Carbonsäureestern zu den entsprechenden alicyclischen Carbonsäureestern die Raum-Zeit-Ausbeute erhöht und/oder die Produktqualität verbessert werden kann, d. h. dass eine geringere Menge an Nebenprodukten erhalten wird, wenn die Hydrierung in mindestens drei hintereinander geschalteten Hydriereinheiten durchgeführt wird, wobei zumindest die beiden ersten Hydriereinheiten in Schlaufenfahrweise, d, h. unter Rückführung eines Teils des jeweiligen Hydrieraustrags, betrieben werden und die Verweilzeit der hintereinandergeschalteten Hydriereinheiten unterschiedlich und im ersten Reaktor niedriger als im zweiten Reaktor ist.

Unter einer Hydriereinheit wird hier und im folgenden Text ein Hydrierreaktor oder mehrere hintereinandergeschalteter Reaktoren oder mehrere parallel zueinandergeschalteten Reaktoren oder eine Reaktorgruppe, die aus parallel und hintereinander geschalteten Reaktoren besteht, verstanden, also ein Reaktor oder eine Reaktoranordnung, die im erfindungsgemäßen Verfahren die Funktion eines Reaktors ausüben kann.

Gegenstand der vorliegenden Erfindung ist deshalb ein Verfahren zur kontinuierlichen Herstellung von alicyclischen Carbonsäureestern durch katalytische Hydrierung der entsprechenden aromatischen Carbonsäureester an festen im Festbett angeordneten Katalysatoren mit einem Wasserstoff-haltigen Gas, welches dadurch gekennzeichnet ist, dass die Hydrierung in mindestens drei hintereinandergeschalteten Hydriereinheiten durchgeführt wird, dass mindestens die ersten beiden Hydriereinheiten in Schlaufenfahrweise betrieben werden und dass zumindest zwei in Reihe geschaltete und in Schlaufenfahrweise betriebene Hydriereinheiten mit unterschiedlichen Verweilzeiten betrieben werden, wobei die Verweilzeit in einer der in Schlaufenfahrweise betriebenen Hydriereinheiten niedriger ist als in einer direkt nachfolgenden in Schlaufenfahrweise betriebenen Hydriereinheit.

Wird im Rahmen der vorliegenden Erfindung von Verweilzeiten gesprochen so sind mittlere Verweilzeiten (reziproke LHSV) gemeint. Diese sind definiert als der Quotient des gegebenen Reaktionsvolumens bzw. des Schüttvolumens des Katalysators (ist bei heterogenen Reaktionssystemen gleich zu setzen) und dem Gesamtvolumenstrom (ohne Recyclestrom) des Einsatzstoffes (des zu hydrierendem Edukts).

Das erfindungsgemäße Verfahren hat den Vorteil, dass durch eine einfache Hintereinanderschaltung von zumindest zwei Schlaufenreaktoren die Raum-Zeit-Ausbeute im Vergleich mit den im Stand der Technik beschriebenen Verfahren erhöht werden kann. Durch die Verwendung von zumindest zwei Schlaufenreaktoren ist zudem eine stabilere und flexiblere Fahrweise möglich. So kann bei Ausfall eines der Schlaufenreaktoren im Notfall unter Umgehung dieses Reaktors die Hydrierung weiter betrieben werden. Durch die Verwendung der zumindest zwei Schlaufenreaktoren wird also auch eine höhere Ausfallsicherheit gewährleistet. Zudem wird eine einfachere Wartung des Reaktorsystems und eine höhere Standzeit der Katalysatoren erreicht.

Das erfindungsgemäße Verfahren wird nachfolgend beispielhaft beschrieben, ohne dass die Erfindung auf die beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen erfassen, die explizit erwähnt sind, sondern auch alle Teilbereiche und Teilgruppen von Verbindungen, die durch Weglassen von einzelnen Werten (Bereichen) oder Verbindungen erhalten werden können.

Das erfindungsgemäße Verfahren zur kontinuierlichen Herstellung von alicyclischen Carbonsäureestern durch katalytische Hydrierung der entsprechenden aromatischen Carbonsäureester an festen, im Festbett angeordneten Katalysatoren mit einem wasserstoffhaltigen Gas, zeichnet sich dadurch aus, dass die Hydrierung in mindestens drei hintereinandergeschalteten Hydriereinheiten durchgeführt wird, dass mindestens die ersten beiden Hydriereinheiten in Schlaufenfahrweise betrieben werden und dass zumindest zwei in Reihe geschaltete und in Schlaufenfahrweise betriebene Hydriereinheiten mit unterschiedlichen Verweilzeiten betrieben werden, wobei die Verweilzeit in einer der in Schlaufenfahrweise betriebenen Hydriereinheiten niedriger ist als in einer direkt nachfolgenden in Schlaufenfahrweise betriebenen Hydriereinheit. Es kann vorteilhaft sein, wenn alle Hydriereinheiten in Schlaufenfahrweise betrieben werden. Ebenso vorteilhaft kann es sein, wenn die letzte Hydriereinheit im geraden Durchgang betrieben wird.

Das Verhältnis der Verweilzeiten in den in Reihe hintereinandergeschalteten Schlaufenreaktoren beträgt vorzugsweise von 0,01 bis kleiner 1, bevorzugt von 0,1 bis 0,9 und besonders bevorzugt von 0,2 bis 0,5. Die Verweilzeiten werden vorzugsweise so eingestellt, dass in dem ersten der hintereinandergeschalteten Schlaufenreaktoren ein Umsatz von 40 bis 90 %, bevorzugt von 60 bis 90 % und im zweiten Schlaufenreaktor ein Umsatz von 2 bis 60 %, bevorzugt von 2 bis 40 % bezogen auf die Ausgangskonzentration der zu hydrierenden Verbindung am Eingang des jeweiligen Reaktors erzielt wird.

Durch die Verwendung von zwei in Reihe geschalteten Schlaufenreaktoren mit unterschiedlicher Verweilzeit und einer Fahrweise im Bereich der besonders bevorzugten Umsätze, wird bei der Hydrierung von Diisononylphthalaten oder Didecylphthalaten (Diisodecylphthalaten) eine optimale Ausnutzung des Katalysatorvolumens erreicht.

Es versteht sich von selbst, dass mehr als zwei in Reihe geschaltete Schlaufenreaktoren in dem erfindungsgemäßen Verfahren vorhanden sein können. Auch ist es in dem erfindungsgemäßen Verfahren bei der Anwesenheit von mehr als zwei hintereinandergeschalteten Schlaufenreaktoren möglich, dass einer oder mehrere dieser Reaktoren so gefahren werden, dass hintereinandergeschaltete Schlaufenreaktoren nicht mit unterschiedlichen Verweilzeiten gefahren werden, bei denen der vordere Reaktor mit einer niedrigeren Verweilzeit als der nachfolgende Reaktor betrieben wird, solang zumindest zwei in Reihe geschaltete Schlaufenreaktoren vorhanden sind, bei denen der vordere Reaktor mit einer niedrigeren Verweilzeit als der nachfolgende Reaktor betrieben wird.

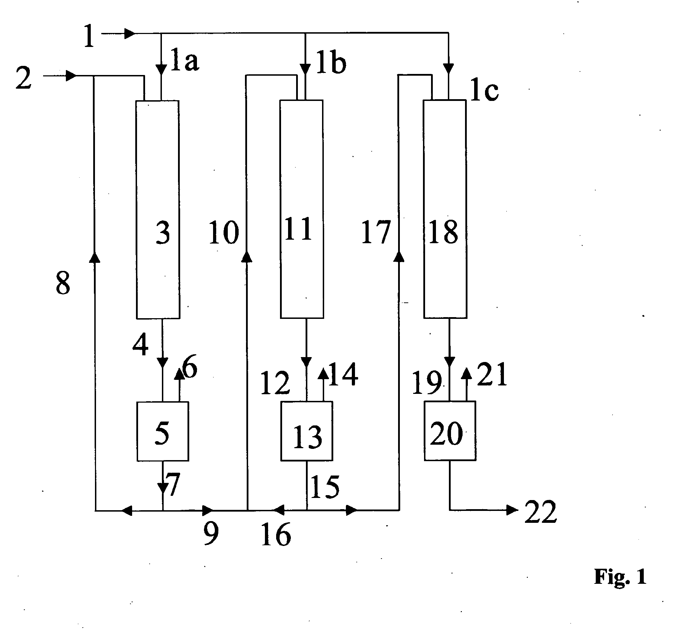

Im erfindungsgemäßen Verfahren kann eine aromatischer Carbonsäureester oder ein Gemisch aromatischer Carbonsäureester in der Flüssigphase oder Flüssig/Gas-Mischphase kontinuierlich an einem im Festbett angeordneten Katalysator in mindestens drei hintereinandergeschalteten Hydriereinheiten mit Wasserstoff zu dem/den entsprechenden alicyclischen Carbonsäureester hydriert werden. Eine Variante des erfindungsgemäßen Verfahrens mit drei Hydriereinheiten ist als Blockschema in Fig. 1 dargestellt. Es sei betont, dass die hier dargestellte Variante sinngemäß auch für Verfahren mit mehr als drei Hydriereinheiten gilt.

In der in Fig. 1 dargestellten Ausführungsform der Erfindung werden die ersten beiden Hydriereinheiten in Schlaufenfahrweise und die dritte Hydriereinheit im geraden Durchgang betrieben. Es sind weitere Ausführungsformen möglich, bei denen alle drei Hydriereinheiten in Schlaufenfahrweise betrieben werden oder bei denen mehr als drei Hydriereinheiten vorhanden sind. Wird die Hydrierung in einer Hydrieranlage mit mehr als drei Hydriereinheiten durchgeführt, gilt gemäß der Erfindung, dass die ersten beiden Hydriereinheiten in Schlaufenfahrweise betrieben werden und die folgenden Hydriereinheiten wahlweise in Schlaufenfahrweise oder im geraden Durchgang betrieben werden können.

In der in Fig. 1 dargestellten Variante des erfindungsgemäßen Verfahrens wird jeder einzelne Reaktor mit Hydriergas beschickt. Um den Wasserstoffverbrauch und die durch die Abgasströme bedingten Austragsverluste zu minimieren, kann es zweckmäßig sein, das Abgas einer Hydriereinheit als Hydriergas für eine andere Hydriereinheit zu verwenden. Beispielweise kann in einem Verfahren wie in Fig. 1 dargestellt das Abgas (6) aus der ersten Hydriereinheit (3) anstelle des Hydriergases (1b) in die zweite Hydriereinheit (11) und das Abgas (14) der zweiten Hydriereinheit (11) anstelle des Hydriergases (1c) in die dritte Hydriereinheit (18) eingespeist werden. In diesem Falle strömen flüssige Edukt/Produkt-Phase und Hydriergas in gleicher Reihenfolge durch die Reaktoren. Ebenso kann es zweckmäßig sein, Hydriergas und Edukt/Produkt-Phase in entgegengesetzter Richtung durch die Reaktoren strömen zu lassen. In diesem Falle wird frisches Hydriergas in den letzten Reaktor eingeleitet und Abgas aus dem ersten Reaktor abgeleitet. Weiterhin können zwei oder mehrere Reaktoren ein gemeinsames Hydriergassystem besitzen und andere Reaktoren davon getrennt mit Hydriergas beschickt werden. Bei der Verwendung des Abgases eines Reaktors als Hydriergas eines anderen Reaktors kann, falls gewünscht, der Druckverlust durch Zwischenkompression ausgeglichen werden.

Vorzugsweise werden die Abgasmengen bzw. Gasströme so eingestellt, dass in allen Reaktoren eine gute Fluiddynamik, also eine geringe Wandströmung und eine hohe Grenzfläche zum Stoffaustausch, vorliegt.

Mengen an Katalysatorgiften, wie beispielsweise Kohlenmonoxid oder Schwefelwasserstoff enthalten, eingesetzt werden. Die Verwendung von Inertgasen ist optional, bevorzugt wird Wasserstoff in einer Reinheit größer 95 %, insbesondere größer 98 % eingesetzt. Inertgasanteile können beispielsweise Stickstoff oder Methan sein. Vorzugsweise ist in den Hydriereinheiten soviel Wasserstoff vorhanden, dass dieser im Überschuss, insbesondere in einem Überschuss von 200 %, bevorzugt in einem Überschuss von 5 bis 100 % und besonders bevorzugt in einem Überschuss von 10 bis 50 % bezogen auf die stöchiometrische Menge, die zur Erzielung des in der Hydriereinheit möglichen bzw. gewünschten Umsatzes benötigt wird, vorliegt. Ohne das Einstellen eines ausreichenden Überschusses an Wasserstoff gelingt die Hydrierung der aromatischen Bindungen nur unvollkommen, was zu Ausbeuteverlusten führt.

Nach dem erfindungsgemäßen Verfahren können aromatische Carbonsäureester, wie aromatische Mono-, Di- oder Polycarbonsäureester, insbesondere deren Alkylester, zu den entsprechenden alicyclischen Carbonsäureverbindungen umgesetzt werden. Dabei können als aromatische Di- oder Polycarbonsäurederivate sowohl Vollester als auch Partialester mit dem erfindungsgemäßen Verfahren hydriert werden. Unter Vollester wird eine Verbindung verstanden, bei der alle Säuregruppen verestert sind. Partialester sind Verbindungen mit mindestens einer freien Säuregruppe (oder gegebenenfalls einer Anhydridgruppe) und mindestens einer Estergruppe. Werden im erfindungsgemäßen Verfahren Polycarbonsäureester eingesetzt, so enthalten diese bevorzugt 2, 3 oder 4 Esterfunktionen.

Im erfindungsgemäßen Verfahren können als aromatischen Di- oder Polycarbonsäureester bevorzugt Benzol-, Diphenyl-, Naphthalin-, Diphenyloxid- oder Anthracenpolycarbonsäureester, eingesetzt werden. Die durch das erfindungsgemäße Verfahren erhaltenen alicyclischen Di- oder Polycarbonsäureester bestehen aus einem oder mehreren, gegebenenfalls durch eine C-C-Bindung verknüpfte oder ankondensierte C₆-Ringen.

In einer bevorzugten Ausführungsform betrifft die vorliegende Erfindung ein Verfahren zur Hydrierung der 1,2-, 1,3- oder 1,4-Benzoldicarbonsäureester, und/oder der 1,2,3-, 1,2,4- oder 1,3,5-Benzoltricarbonsäureester, d. h. es werden die Isomere der 1,2-, 1,3- oder 1,4-Cyclohexandicarbonsäureester, oder der 1,2,3-, 1,3,5- oder 1,2,4-Cyclohexantricarbonsäureester erhalten.

Im erfindungsgemäßen Verfahren können beispielsweise Ester folgender aromatischer Carbonsäuren eingesetzt werden: 1,2-Naphthalindicarbonsäure, 1,3-Naphthalindicarbonsäure, 1,4-Naphthalindicarbonsäure, 1,5-Naphthalindicarbonsäure, 1,6-Naphthalindicarbonsäure, 1,7-Naphthalindicarbonsäure, 1,8-Naphthalindicarbonsäure, Phthalsäure (Benzol-1,2-dicarbonsäure), Isophthalsäure (Benzol-1,3-dicarbonsäure), Terephthalsäure (Benzol-1,4-dicarbonsäure), Benzol-1,2,3-tricarbonsäure, Benzol-1,2,4-tricarbonsäure (Trimellitsäure), Benzol-1,3,5-tricarbonsäure (Trimesinsäure), Benzol-1,2,3,4-tetracarbonsäure. Weiterhin können Ester eingesetzt werden, die aus den genannten Säuren durch Substitution eines oder mehrerer am aromatischen Kern gebundenen Wasserstoffatome durch Alkyl-, Cycloalkyl- oder Alkoxyalkylgruppen entstehen.

Als aromatische Monocarbonsäureester können in dem erfindungsgemäßen Verfahren Ester von z. B. Benzoesäure, 1-Naphthoesäure oder 2-Naphthoesäure eingesetzt werden. Weiterhin können Monocarbonsäureester eingesetzt werden, die aus den genannten Monocarbonsäuren durch Substitution eines oder mehrerer am aromatischen Kern gebundenen Wasserstoffatome durch Alkyl-, Cycloalkyl- oder Alkoxyalkylgruppen entstehen.

In dem erfindungsgemäßen Verfahren werden die aromatischen Carbonsäureester der vorgenannten aromatischen Carbonsäuren eingesetzt. Die Alkoholkomponente der bevorzugt eingesetzten aromatischen Carbonsäureester besteht bevorzugt aus verzweigten oder linearen (unverzweigten) Alkyl-, Cycloalkyl- oder Alkoxyalkylgruppen mit 1 bis 25 Kohlenstoffatomen, vorzugsweise 3 bis 15, besonders bevorzugt 8 bis 13 C-Atomen und ganz besonders bevorzugt 9 oder 10 C-Atomen. Die Alkoxyalkylgruppen mit 1 bis 25 Kohlenstoffatomen, vorzugsweise 3 bis 15, besonders bevorzugt 8 bis 13 C-Atomen und ganz besonders bevorzugt 9 oder 10 C-Atomen. Die Alkoholkomponente kann eine oder mehrere Hydroxy-Gruppen aufweisen. Sind in einem Molekül mehr als eine Carboxygruppe vorhanden, so können die Alkoholkomponenten in einem Molekül eines eingesetzten aromatischen Polycarbonsäureesters gleich oder unterschiedlich sein, d. h. sie können gleiche oder verschiedene Isomeren oder Kettenlängen besitzen. Selbstverständlich können auch Isomere bezüglich des Substitutionsmusters des aromatischen Systems in Form eines Gemisches eingesetzt werden, z. B. ein Gemisch aus Phthalsäureester und Terephthalsäureester.

Im erfindungsgemäßen Verfahren können als Ester einer aromatischen Di- oder Polycarbonsäure beispielsweise folgende Verbindungen eingesetzt werden: Terephthalsäuremonomethylester, Terephthalsäuredimethylester, Terephthalsäurediethylester, Terephthalsäuredi-n-propylester, Terephthalsäuredibutylester, Terephthalsäurediisobutylester, Terephthalsäuredi-tert.-butylester, Terephthalsäuremonoglykolester, Terephthalsäurediglykolester, Terephthalsäure-düsoheptylester, Terephthalsäure-n-octylester, Terephthalsäurediisooctylester, Terephthalsäuredi-2-ethylhexylester, Terephthalsäuredi-n-nonylester, Terephthalsäurediisononylester, Terephthalsäuredi-n-decylester, Terephthalsäurediisodecylester, Terephthalsäuredipropylheptylester, Terephthalsäuredi-n-undecylester, Terephthalsäurediisododecylester, Terephthalsäure-ditridecylester, Terephthalsäuredi-n-octadecylester, Terephthalsäurediisooctadecylester, Terephthalsäuredi-n-eicosylester, Terephthalsäuremonocyclohexylester; Phthalsäuremonomethylester, Phthalsäuredimethylester, Phthalsäuredi-n-propylester, Phthalsäuredi-n-butylester, Phthalsäurediisobutylester, Phthalsäuredi-tert.-butylester, Phthalsäuremonoglykolester, Phthalsäurediglykolester, Phthalsäurediisoheptylester, Phthalsäuredi-n-octylester, Phthalsäurediisooctylester, Phthalsäurediethylhexylester, Phthalsäuredi-n-nonylester, Phthalsäurediisononylester, Phthalsäuredi-n-decylester, Phthalsäuredi-2-propylheptylester, Phthalsäurediisodecylester, Phthalsäuredi-n-undecylester, Phthalsäurediisoundecylester, Phthalsäureditridecylester, Phthalsäuredi-n-octadecylester, Phthalsäurediisooctadecylester, Phthalsäuredi-n-eicosylester, Phthalsäuremonocyclohexylester; Phthalsäuredicyclohexylester, Isophthalsäuremonomethylester, Isophthalsäuredimethylester, Isophthalsäuredimethylester, Isophthalsäurediethylester, Isophthalsäuredi-n-propylester, Isophthalsäurediisoheptylester, Isophthalsäuredi-n-octylester, Isophthalsäurediisooctylester, Isophthalsäuredi-2-ethylhexylester, Isophthalsäuredi-n-nonylester, Isophthalsäurediisononylester, Isophthalsäuredi-n-decylester, Isophthalsäurediisodecylester, Isophthalsäuredipropylheptylester Isophthalsäuredi-n-undecylester, Isophthalsäurediisododecylester, Isophthalsäuredi-n-dodecylester, Isophthalsäureditridecylester, Isophthalsäuredi-n-octadecylester, Isophthalsäurediisooctadecylester, Isophthalsäuredi-n-eicosylester, Isophthalsäuremonocyclohexylester.

Im erfindungsgemäßen Verfahren können als Ester der Monocarbonsäuren z. B. Benzoate von Diolen, wie beispielsweise Glycoldibenzoat, Diethylenglycoldibenzoat, Triethylenglycoldibenzoat oder Dipropylenglycoldibenzoat, aber auch Benzoesäurealkylester, wie z. B. Decyl- oder Isodecylbenzoat, Nonyl- oder Isononylbenzoat, Octyl- oder Isooctylbenzoat, 2-Ethylhexylbenzoat oder Tridecyl- oder Isotridecylbenzoat eingesetzt werden.

In dem erfindungsgemäßen Verfahren können auch Gemische aus zwei oder mehreren Carbonsäureestern eingesetzt werden. Solche Gemische können beispielsweise auf folgenden Wegen erhalten werden:
a) Eine Di- oder Polycarbonsäure wird mit einem Alkohol derart partiell verestert, dass Voll- und Partialester nebeneinander vorliegen.
b) Ein Gemisch von mindestens zwei Carbonsäuren wird mit einem Alkohol verestert, wobei ein Gemisch von mindestens zwei Vollestern entsteht.
c) Eine Di- oder Polycarbonsäure wird mit einem Alkoholgemisch versetzt, wobei ein entsprechendes Gemisch der Vollester entstehen kann.
d) Eine Di- oder Polycarbonsäure wird mit einem Alkoholgemisch partiell verestert.
e) Ein Gemisch von mindestens zwei Carbonsäuren wird mit einem Alkoholgemisch partiell verestert.
f) Ein Gemisch aus mindestens zwei Di- oder Polycarbonsäuren wird mit einem Alkoholgemisch partiell verestert.

Bei diesen Umsetzungen können an Stelle der Polycarbonsäuren auch die entsprechenden

Bei diesen Umsetzungen können an Stelle der Polycarbonsäuren auch die entsprechenden Anhydride eingesetzt werden.

Großtechnisch werden aromatische Ester, insbesondere die Vollester auf Weg c) häufig aus Alkoholgemischen hergestellt. Entsprechende Alkoholgemische sind beispielsweise:
C₅-Alkoholgemische, hergestellt aus linearen Butenen durch Hydroformylierung und anschließender Hydrierung;
C₅-Alkoholgemische, hergestellt aus Isobuten oder Butengemischen, die lineare Butene und Isobuten enthalten, durch Hydroformylierung und anschließende Hydrierung;
C₆-Alkoholgemische, hergestellt aus einem Penten oder aus einem Gemisch von zwei oder mehreren Pentenen, durch Hydroformylierung und anschließende Hydrierung;
C₇-Alkoholgemische, hergestellt aus Triethylen oder Dipropen oder einem Hexenisomer oder einem sonstigen Gemisch von Hexenisomeren, durch Hydroformylierung und anschließende Hydrierung;
C₈-Alkoholgemische, wie 2-Ethylhexanol (2 Isomere), hergestellt durch Aldolkondensation von n-Butyraldehyd und anschließende Hydrierung;
C₉-Alkoholgemische, hergestellt aus C₄-Olefinen durch Dimerisierung, Hydroformylierung und Hydrierung. Dabei kann zur Herstellung der C₉-Alkohole von Isobuten oder von einem Gemisch linearer Butene oder von Gemischen mit linearen Butenen und Isobuten ausgegangen werden. Die C₄-Olefine können mit Hilfe unterschiedlicher Katalysatoren dimerisiert werden, wie beispielsweise Protonensäuren, Zeolithe, metallorganische Nickelverbindungen oder feste nickelhaltige Kontakte. Die Hydroformylierung der C₈-Olefingemische kann mit Hilfe von Rhodium- oder Kobaltkatalysatoren erfolgen. Es gibt daher ein Vielzahl von technischen C₉₋Alkoholgemischen.
C₁₀-Alkoholgemische, hergestellt aus Tripropylen durch Hydroformylierung und anschließende Hydrierung; 2-Propylheptanol (2 Isomere), hergestellt durch Aldolkondensation von Valeraldehyd und anschließende Hydrierung;
C₁₀-Alkoholgemische, hergestellt aus einem Gemisch von mindestens zwei C₅-Aldehyden durch Aldolkondensation und anschließende Hydrierung;
C₁₃-Alkolgemische, hergestellt aus Dihexen, Hexaethylen, Tetrapropylen oder Tributen durch aus Olefinen bzw. Olefingemischen gewonnen werden, die beispielsweise bei Fischer-Tropsch-Synthesen, bei Dehydrierungen von Kohlenwasserstoffen, Metathesereaktionen, beim Polygasverfahren oder anderen technischen Prozessen anfallen. Darüber hinaus können auch Olefingemische mit Olefinen unterschiedlicher C-Zahlen für die Herstellung von Alkoholgemischen eingesetzt werden.

Im erfindungsgemäßen Verfahren können alle Estergemische, hergestellt aus aromatischen Carbonsäuren und den oben genannten Alkoholgemischen, eingesetzt werden. Erfindungsgemäß werden bevorzugt Ester, hergestellt aus Phthalsäure, Phthalsäureanhydrid oder Benzoesäure und einem Gemisch isomerer Alkohole mit 6 bis 13 C-Atomen, eingesetzt.

Beispiele für technische Phthalate, die im erfindungsgemäßen Verfahren eingesetzt werden können, sind folgende Produkte mit den Handelsnamen:
Vestinol C (Di-n-butylphthalat) (CAS Nr.84-74-2); Vestinol IB (Di-i-butylphthalat) (CAS Nr. 84-69-5); Jayflex DINP (CAS Nr.68515-48-0); Jayflex DIDP (CAS Nr.68515-49-1); Palatinol 9P (68515-45-7), Vestinol 9 (CAS Nr. 28553-12-0); TOTM (CAS Nr. 3319-31-1); Linplast 68-TM , Palatinol N (CAS Nr. 28553-12-0); Jayflex DHP (CAS Nr. 68515-50-4); Jayflex DIOP (CAS Nr. 27554-26-3); Jayflex UDP (CAS Nr. 68515-47-9); Jayflex DIUP (CAS Nr. 85507-79-5); Jayflex DTDP (CAS Nr.68515-47-9); Jayflex L9P (CAS Nr. 68515-45-7); Jayflex L911P (CAS Nr. 68515-43-5); Jayflex L11P (CAS Nr. 3648-20-2); Witamol 110 (CAS Nr. 68515-51-5); Witamol 118 (Di-n-C8-C10-alkylphthalat) (CAS Nr.71662-46-9); Unimoll BB (CAS Nr. 85-68-7); Linplast 1012 BP (CAS Nr. 90193-92-3); Linplast 13XP (CAS Nr.27253-26-5); Linplast 610P (CAS Nr. 68515-51-5); Linplast 68 FP (CAS Nr. 68648-93-1); Linplast 812 HP (CAS Nr. 70693-30-0); Palatinol AH (CAS Nr. 117-81-7); Palatinol 711 (CAS Nr. 68515-42-4); Palatinol 911 (CAS Nr. 68515-43-5); Palatinol 11 (CAS Nr. 3648-20-2); Palatinol Z (CAS Nr.26761-40-0); Palatinol DIPP (CAS Nr. 84777-06-0); Jayflex 77 (CAS Nr. 71888-89-6); Palatinol 10 P (CAS Nr. 533-54-0); Vestinol AH (CAS Nr. 117-81-7).

Es sei darauf hingewiesen, dass bei der Kernhydrierung aromatischer Di- oder Polycarbonsäuren-Ester aus jedem eingesetzten Isomer mindestens zwei stereoisomere Hydrierprodukte entstehen können. Die Mengenverhältnisse der dabei entstandenen Stereoisomeren zueinander hängen vom verwendeten Katalysator und von den Hydrierbedingungen ab. Alle Hydrierprodukte mit beliebigen Verhältnis(sen) der Stereoisomeren zueinander können ohne oder nach einer Auftrennung verwendet werden. In der Regel werden die Hydrierprodukte ohne Auftrennung verwendet.

Im erfindungsgemäßen Verfahren werden feste Hydrierkatalysatoren eingesetzt, die vorzugsweise mindestens ein Metall der achten Nebengruppe des Periodensystems der Elemente enthalten. Vorzugsweise werden als Aktivmetalle der achten Nebengruppe des Periodensystems der Elemente Platin, Rhodium, Palladium, Kobalt, Nickel oder Ruthenium oder ein Gemisch aus zwei oder mehreren davon eingesetzt, wobei insbesondere Ruthenium als Aktivmetall eingesetzt wird.

Neben den bereits genannten Metallen kann zusätzlich mindestens ein Metall der ersten und/oder siebten Nebengruppe des Periodensystems der Elemente in den Katalysatoren enthalten sein. Bevorzugt wird Rhenium und/oder Kupfer eingesetzt.

Der Gehalt der Aktivmetalle, d. h. der Metalle der ersten und/oder siebten und/oder achten Nebengruppe des Periodensystems der Elemente beträgt vorzugsweise 0,1 bis 30 Massen %. Der Edelmetallgehalt, d. h. der Metalle der achten Nebengruppe des Periodensystems der Elemente und der fünften oder sechsten Periode, z. B. Palladium, Ruthenium, berechnet als Metall liegt vorzugsweise im Bereich von 0,1 bis 10 Massen-%, insbesondere im Bereich von 0,8 bis 5 Massen-%, ganz besonders zwischen 1 und 3 Massen-%.

Bevorzugt sind die eingesetzten Katalysatoren Trägerkatalysatoren. Als Träger können beispielsweise folgende Stoffe verwendet werden: Aktivkohle, Siliciumcarbid, Aluminiumoxid, Siliciumoxid, Alumosilikat, Titandioxid, Zirkoniumdioxid, Magnesiumoxid und/oder Zinkoxid oder deren Gemische. Besonders bevorzugt wird ein Katalysator eingesetzt, der einen Titandioxid-Träger aufweist. Zusätzlich können diese Trägermaterialien Alkalimetalle, Erdalkalimetalle und/oder Schwefel enthalten.

Im erfmdungsgemäßen Verfahren werden bevorzugt Ruthenium-Katalysatoren eingesetzt, die in den Patentschriften DE 102 25 565.2 und DE 102 32 868.4 beansprucht werden.

Im erfindungsgemäßen Verfahren bestehen die Hydriereinheiten vorzugsweise jeweils aus einem Hydrierreaktor. Dieser kann ein Rohrreaktor, Rohrbündelreaktor oder vorzugsweise ein Schachtofen sein.

Die einzelnen Reaktoren können adiabatisch, polytrop oder praktisch isotherm, d. h. mit einem Temperaturanstieg von typischerweise kleiner als 10 °C betrieben werden. Dabei werden insbesondere die in Schlaufenfahrweise betriebenen Reaktoren bevorzugt quasi isotherm gefahren, vorzugsweise mit einem Temperaturanstieg kleiner 10 °C besonders bevorzugt kleiner 5 °C betrieben.

Das erfindungsgemäße Verfahren wird bevorzugt in der Flüssig/Gas-Mischphase oder Flüssigphase in Dreiphasenreaktoren im Gleichstrom durchgeführt, wobei das Hydriergas in an sich bekannter Weise im flüssigen Edukt/Produktstrom verteilt wird. Im Interesse einer gleichmäßigen Flüssigkeitsverteilung, einer verbesserten Reaktionswärmeabfuhr und/oder einer hohen Raum-Zeit-Ausbeute werden die in Schlaufenfahrweise betriebenen Reaktoren vorzugsweise mit hohen Flüssigkeitsbelastungen von 10 bis 400, bevorzugt von 20 bis 200 und besonders bevorzugt von 40 bis 150 m³ pro m² Querschnitt des leeren Reaktors und Stunde gefahren.

Die Flüssigkeitsbelastungen können in den in Schlaufenfahrweise betriebenen Reaktoren gleich oder verschieden sein. Vorzugsweise ist die Flüssigkeitsbelastung im ersten Reaktor am größten und nimmt in den nachfolgenden in Schlaufenfahrweise betriebenen Reaktoren ab. In einer Anlage gemäß der Erfindung mit zwei hintereinandergeschalteten Schlaufenreaktoren liegt die Flüssigbelastung im ersten Reaktor bevorzugt im Bereich von 20 bis 200, insbesondere im Bereich von 40 bis 150 m³/(m²*h) und im zweiten Reaktor bevorzugt im Bereich von 20 bis 180, insbesondere im Bereich von 40 bis 140 m³/(m²*h).
Die Belastung des im geraden Durchgang betriebenen Reaktors beträgt vorzugsweise von 2 bis 100 m³/(m²*h), insbesondere von 10 bis 80 m³/(m^{2*}h).

Die Hydrierung kann in Ab- oder vorzugsweise in Anwesenheit eines Lösungsmittels durchgeführt werden. Als Lösemittel können alle Flüssigkeiten eingesetzt werden, die mit dem Edukt und Produkt eine homogene Lösung bilden, sich unter Hydrierbedingungen inert verhalten und sich leicht vom Produkt abtrennen lassen. Das Lösemittel kann auch ein Gemisch mehrerer Stoffe sein und gegebenenfalls Wasser enthalten.

Beispielsweise können folgende Stoffe als Lösemittel eingesetzt werden: Geradkettige oder cyclische Ether, wie beispielsweise Tetrahydrofuran oder Dioxan, sowie aliphatische Alkohole, in denen der Alkylrest 1 bis 13 Kohlenstoffatome aufweist. Bevorzugt als Lösemittel verwendbare Alkohole sind Isopropanol, n-Butanol, Isobutanol, n-Pentanol, 2-Ethylhexanol, Nonanole, technische Nonanolgemische, Decanol, technische Decanolgemische, Tridecanole. Die Verwendung von Alkoholen ist nur dann bevorzugt, wenn die zur Hydrierung vorgesehenen Carbonsäurederivate Ester sind. Bei Einsatz von Alkoholen als Lösemittel kann es zweckmäßig sein, denjenigen Alkohol oder dasjenige Alkoholgemisch zu verwenden, das bei der Verseifung des Produkts entstehen würde. Dadurch würde die Nebenproduktbildung durch Umesterung ausgeschlossen. Ein weiteres bevorzugtes Lösemittel ist das Hydrierprodukt selbst.

Durch die Verwendung eines Lösemittels kann die Aromatenkonzentration im Reaktorzulauf begrenzt werden, wodurch eine bessere Temperaturkontrolle im Reaktor erreicht werden kann. Dies kann eine Minimierung von Nebenreaktionen und somit eine Erhöhung der Produktausbeute zur Folge haben. Bevorzugt liegt der Aromatengehalt im Reaktorzulauf zwischen 1 und 70 %, Der gewünschte Konzentrationsbereich kann bei denjenigen Reaktoren, die in Schlaufenfahrweise betrieben werden, durch das Kreislaufverhältnis (Mengenverhältnis von rückgeiührten Hydrieraustrag zu Edukt) eingestellt werden. Die Aromatenkonzentrationen im Reaktorzulauf (Gemisch aus Frisch-Edukt oder Hydrieraustrag des vorigen Reaktors und aus Kreislaufstrom) nehmen vom ersten bis zum letzten Reaktor bevorzugt ab. Beispielweise liegen in einer Anlage nach Fig. 1 die Aromatenkonzentration im Zulauf zum ersten Reaktor (3) im Bereich von 70 bis 5 Massen-%, im Zulauf zum zweiten Reaktor (11) im Bereich von 40 bis 2 Massen-% und im Zulauf zum dritten Reaktor (18) im Bereich von 20 bis 1 Massen-%.

Das erfindungsgemäße Verfahren wird bevorzugt in einem Druckbereich von 0,3 bis 30 MPa, insbesondere von 1,5 bis 20 MPa, ganz besonders bevorzugt von 5 bis 20 MPa durchgeführt. Der Druck kann in den einzelnen Reaktoren gleich oder verschieden sein. Vorzugsweise sind die Drücke gleich oder annähernd gleich.

Die Hydriertemperaturen betragen bevorzugt von 50 bis 250 °C, vorzugsweise von 80 bis 200 °C. Die Hydriertemperaturen können in einzelnen Reaktoren gleich oder verschieden sein.

Das Hydrierprodukt, das bei der erfindungsgemäßen Hydrierung eines aromatischen Carbonsäureesters, insbesondere eines aromatischen Di- oder Polycarbonsäureesters oder eines Gemisches aromatischer Di- oder Polycarbonsäureester nach dem erfindungsgemäßen Verfahren entsteht, hat vorzugsweise einen Gehalt an alicyclischen Carbonsäuren-Estern von über 96 Massen-%, insbesondere von über 98 Massen-%, ganz besonders bevorzugt von über 99 Massen-%. Dieses Gemisch kann direkt oder nach einer Reinigung eingesetzt werden. Die Abtrennung von Nebenprodukten kann beispielsweise durch Destillation oder durch Strippen mit einem Inertgas wie Stickstoff oder Wasserdampf erfolgen. Bevorzugt werden geringe Mengen an Leichtsieder durch Strippen mit Wasserdampf im Temperaturbereich von 120 °C bis 240 °C, insbesondere im Bereich von 150 bis 200 °C und bei einem Druck von 5 kPa bis 10 kPa abgetrennt. Anschließend kann durch Verringern des Druckes auf unter 5 kPa das Produkt getrocknet werden.

Als Produkte werden mit dem erfindungsgemäßen Verfahren Gemische erhalten, die alicyclische Carbonsäureester und besonders bevorzugt alicyclische Di- oder Polycarbonsäureester aufweisen.

Ein weiterer Gegenstand der vorliegenden Erfindung ist die Verwendung der erfindungsgemäß hergestellten Gemische von alicyclischen Carbonsäureester als Weichmacher in Kunststoffen. Bevorzugte Kunststoffe sind PVC, Homo- und Copolymere auf Basis von Ethylen, Propylen, Butadien, Vinylacetat, Glycidylacrylat, Glycidylmethacrylat, oder unverzweigten Alkoholen mit einem bis zehn Kohlenstoffatome(n), Styrol, Acrylnitril, Homo- oder Copolymere von cyclischen Olefinen.
Als Vertreter der obigen Gruppen seien beispielsweise folgende Kunststoffe genannt:
Polyacrylate mit gleichen oder verschiedenen Alkylresten mit 4 bis 8 C-Atomen, gebunden am Sauerstoffatom der Estergruppe, insbesondere mit dem n-Butyl-, n-Hexyl-, n-Octyl- und 2-Ethylhexylrest und Isononylrest, Polymethacrylat, Polymethylmethacrylat, Methylacrylat-Butylacrylat-Copolymere, Methylmethacrylat-Butylmethacrylat-Copolymere, EthylenVinylacetat-Copolymere, chloriertes Polyethylen, Nitrilkautschuk, Acrylnitril-Butadien-StyrolCopolymere, Ethylen-Propylen-Copolymere, Ethylen-Propylen-Dien-Copolymere, Styrol-Acrylnitril-Copolymere, Acrylnitril-Butadien-Kautschuk, Styrol-Butadien-Elastomere, Methylmethacrylat-Styrol-Butadien-Copolymere und/oder Nitrocellulose.

Darüber hinaus können die erfindungsgemäß hergestellten alicyclischen Carbonsäureester zur Modifizierung von Kunststoffmischungen, beispielsweise der Mischung eines Polyolefins mit einem Polyamid, eingesetzt werden. Gemische aus Kunststoffen und den erfindungsgemäß hergestellten alicyclischen Polycarbonsäureestern sind ebenfalls Gegenstand der vorliegenden Erfindung. Geeignete Kunststoffe sind die bereits genannten Verbindungen. Solche Gemische enthalten bevorzugt mindestens 5 Massen-%, besonders bevorzugt 10-80 Massen-%, ganz besonders bevorzugt 20-70 Massen-% der alicyclischen Polycarbonsäureester.

Gemische aus Kunststoffen, insbesondere PVC, die einen oder mehrere der erfindungsgemäß hergestellten alicyclischen Polycarbonsäureester enthalten, können beispielsweise in folgenden Produkten enthalten sein, bzw. zu deren Herstellung verwendet werden:
Schläuchen, Kabeln, Drahtummantelungen, Isolierbändern, im Fahrzeug- und Möbelbau, Plastisole, in Bodenbelägen, medizinische Artikel, Lebensmittelverpackungen, Dichtungen, Folien, Verbundfolien, Platten, Kunstleder, Spielzeug, Tapeten, Verpackungsbehälter, Klebebandfolien, Bekleidung, Beschichtungen, Beschichtungen von Geweben, Schuhen, Unterbodenschutz, Nahtabdichtungen, Modelliermassen oder Bällen.
Neben den obengenannten Anwendungen können die erfindungsgemäß hergestellten alicyclischen Carbonsäureester als Schmierölkomponente, als Bestandteil von Kühlflüssigkeiten und Metallbearbeitungsflüssigkeiten verwendet werden. Ebenso können sie als Komponente in Farben, Lacken, Tinten und Klebstoffen eingesetzt werden.
Das erfindungsgemäße Verfahren kann in verschiedenen Ausführungsformen durchgeführt werden. Eine bevorzugte Ausführungsformen der vorliegenden Erfindung ist als Blockschema in der Figur Fig. 1 beispielhaft dargestellt. Dieses Schema weist drei Reaktoren bzw. Reaktoreinheiten auf, von denen zwei in Schlaufenfahrweise betrieben werden. Es versteht sich von selbst, dass das erfindungsgemäße Verfahren auch mit mehr als drei Reaktoren (bzw. Reaktoreinheiten) durchgeführt werden kann oder dass alle drei Reaktoren in Schlaufenfahrweise betrieben werden können.

In der Variante des erfmdungsgemäßen Verfahrens nach Fig. 1 werden Wasserstoff (la), Edukt (2) und ein Teil (8) des flüssigen Hydrieraustrags (7) aus Reaktor (3) in die Hydriereinheit (3) eingespeist. Der Hydrieraustrag (4) aus der Hydriereinheit (3) wird in der Blase (5) in Abgas (6) und Flüssigphase (7) getrennt. Ein Teil (9) des Stroms (7) wird zusammen mit dem Teil (16) der Flüssigphase (15) aus der zweiten Hydriereinheit (11) und Wasserstoff (1b) in die Hydriereinheit (11)geleitet. Der Hydrieraustrag (12) aus der Hydriereinheit (11) wird in der Blase (13) in Abgas (14) und Flüssigphase (15) getrennt. Ein Teil (17) des Stroms (15) wird zusammen mit Wasserstoff (1c) in die Hydriereinheit (18) eingespeist. Der Hydrieraustrag (19) aus der Hydriereinheit (18) wird in der Blase (20) in Abgas (21) und Rohprodukt (22) getrennt. Rohprodukt (22) wird entweder als solches oder nach Aufreinigung in einer nicht dargestellten Anlage verwendet.

Die vorliegende Erfindung wird in den nachfolgenden Beispielen beispielhaft beschrieben, ohne dass die Erfindung auf die in den Beispielen angegebenen Ausführungsformen beschränkt sein soll.

### Beispiele

Der Hydrierreaktor war ein Rohrreaktor und wurde wahlweise im geraden Durchgang oder in Schlaufenfahrweise kontinuierlich betrieben. Bei allen Versuchen strömten die flüssige Phase und das Hydriergas im Gleichstrom von oben nach unten.

Der Rohrreaktor war mit 1350 ml Ruthenium-Katalysator (1 % Ru/TiO₂ gefüllt. Dieser Katalysator wurde aus dem TiO₂-Träger Aerolyst 7711 und einer wässrigen Rutheniumnitrat-Lösung, wie in DE 102 32 868.4 beschrieben, hergestellt. Der Katalysator bestand aus zylindrischen Strangextrudaten mit dem Kreisdurchmesser von 1,5 mm und einer Länge von 4 bis 6 mm.

In beiden Versuchen wurde Diisononylphthalat, abgekürzte Schreibweise DINP, der Oxeno Olefinchemie GmbH mit der Markenbezeichnung Vestinol 9 eingesetzt. Als Hydriergas wurde Wasserstoff in einer Reinheit von über 99,9 % eingesetzt.

Bei beiden Beispielen ist der flüssige Hydrieraustrag einer Hydrierstufe das Einsatzprodukt der nächsten Hydrierstufe. Die einzelnen Hydrierstufen wurden nacheinander im gleichen Reaktor mit dem gleichen Katalysator und gleicher Katalysatormenge durchgeführt. Der Hydrieraustrag der ersten Stufe wurde nach Erreichen des quasistationären Gleichgewichts gesammelt und für die zweite Stufe verwendet. Im Beispiel 1 wurde zusätzlich der Hydrieraustrag der zweiten Stufe als Einsatzmaterial der dritten Stufe gesammelt. Zur besseren Vergleichbarkeit war der Druck, die Reaktionstemperatur und die Abgasmenge in allen Hydrierschritten gleich.

### Beispiel 1 (erfindungsgemäß)

Die Hydrierung wurde in drei Stufen durchgeführt. In den ersten beiden Stufen wurde der Reaktor in Schlaufenfahrweise und in der dritten Stufe im geraden Durchgang betrieben. Die Betriebsparameter und die Massenströme von Beispiel 1 wurden in Tabelle 1 zusammengestellt.

**Tabelle 1:**

| | 1. Stufe | 2. Stufe | 3. Stufe |
|---|---|---|---|
| Temperatur (°C) | 100 | 100 | 100 |
| Druck (MPa) | 10 | 10 | 10 |
| Abgas (Nl/h) | 50 | 50 | 50 |
| Kreislaufinenge (l/h) | 30 | 30 | 0 |
| Zulauf (l/h) | 9,54 | 3,18 | 1,05 |
| DINP-Konzentration im Zulauf (%) (bezogen auf Frisch-DINP oder Hydrieraustrag des vorherigen Reaktors) | 100 | 59,7 | 19,9 |
| Hydrieraustrag (l/h) * | 9,592 | 3,197 | 1,053 |
| DINP-Konzentration im Hydrieraustrag (%) | 59,7 | 19,9 | <0,05 |
| LHSV (h⁻¹) ** | 7,06 | 2,35 | 0,78 |

| | | | |
|---|---|---|---|
| * Volumen des Hydrieraustrags, berechnet unter Zugrundelegung einer Dichte von 0,975 g/l und unter Vernachlässigung der Abgasverluste ** LHSV : Liter Frisch-DINP oder Liter Hydrieraustrag aus dem vorigen Reaktor je Liter Katalysator je Stunde | | | |

Das Zielprodukt (Hydrieraustrag der dritten Stufe) hatte eine Reinheit von über 99,5 Massen-%. Der DINP-Umsatz war praktisch quantitativ.

Unter Berücksichtigung, dass der Hydrieraustrag einer Stufe das Einsatzmaterial der nächsten Stufe ist, ergäben sich, ausgehend von 9,54 l/h Frisch-DINP, für eine kontinuierliche Hydrierung unter Berücksichtigung der unterschiedlichen Dichten von DINP bzw. DINCH folgende Zulaufströme (ohne Rückrührströme) zu den Reaktoren:
Erster Reaktor : 9,54 l/h
Zweiter Reaktor: 9,592 l/h
Dritter Reaktor : 9,643 l/h
Unter Beibehaltung der in Tabelle 1 ausgewiesenen LHSV ergäben sich für die gerade genannten Volumenströme folgende Katalysatormengen:
   Erster Reaktor: 1,351
   Zweiter Reaktor : 4,071
   Dritter Reaktor: 12,361

Die (Gesamt-)Katalysatormenge in den beiden Schlaufenreaktoren entsprach demnach 5,432 1. Bei der Zuführung von 9,54 1/h Frisch-DINP zum ersten Reaktor ergab sich über die beiden Schlaufenreaktoren eine Gesamt-LHSV von 1,75 h⁻¹.

### Beispiel 2 (Vergleich)

Die Hydrierung wurde in zwei Stufen durchgeführt. In der ersten Stufe wurde der Reaktor in Schlaufenfahrweise und in der zweiten im geraden Durchgang betrieben.

Die Betriebsparameter und die Massenströme von Beispiel 2 werden in Tabelle 2 aufgelistet.

**Tabelle 2:**

| | 1. Stufe | 2. Stufe |
|---|---|---|
| Temperatur (°C) | 100 | 100 |
| Druck (MPa) | 10 | 10 |
| Abgas(Nl/h) | 50 | 50 |
| Kreislaufmenge (l/h) | 30 | 0 |
| Zulauf (l/h) | 1,59 | 1,05 |
| DINP-Konzentration im Zulauf (%) (bezogen auf Frisch-DINP oder Hydrieraustrag des vorherigen Reaktors) | 100 | 19,9 |
| Hydrieraustrag (l/h) | 1,625 | 1,053 |
| DINP-Konzentration im Hydrieraustrag (%) | 19,9 | < 0,05 |
| LHSV (h⁻¹) | 1,18 | 0,78 |

Bei der Hydrierung eines Rein-DINP auf einen Restgehalt von 19,9 % erfolgte die Hydrierung in zwei hintereinander geschalteten Schlaufenreaktoren (Beispiel 1) mit einer LHSV von 1,75 h⁻¹, bei der Hydrierung zum gleichen Restgehalt unter Verwendung nur eines Schlaufenreaktors (Beispiel 2) dagegen betrug die LHSV 1,18 h⁻¹. Damit wurde gezeigt, dass das erfindungsgemäße Verfahren gegenüber einem herkömmlichen Verfahren eine höhere Raum-Zeit-Ausbeute aufweist.

## Patentansprüche

1. Verfahren zur kontinuierlichen Herstellung von alicyclischen Carbonsäureestern durch katalytische Hydrierung der entsprechenden aromatischen Carbonsäureester an festen im Festbett angeordneten Katalysatoren mit einem wasserstoffhaltigen Gas,
**dadurch gekennzeichnet,**
**dass** die Hydrierung in mindestens drei hintereinandergeschalteten Hydriereinheiten durchgeführt wird, dass mindestens die ersten beiden Hydriereinheiten in Schlaufenfahrweise betrieben werden und dass zumindest zwei in Reihe geschaltete und in Schlaufenfahrweise betriebene Hydriereinheiten mit unterschiedlichen Verweilzeiten betrieben werden, wobei die Verweilzeit in einer der in Schlaufenfahrweise betriebenen Hydriereinheiten niedriger ist als in einer direkt nachfolgenden in Schlaufenfahrweise betriebenen Hydriereinheit.

2. Verfahren nach Anspruch 1,
**dadurch gekennzeichnet,**
**dass** alle Hydriereinheiten in Schlaufenfahrweise betrieben werden.

3. Verfahren nach Anspruch 1,
**dadurch gekennzeichnet,**
**dass** die letzte Hydriereinheit im geraden Durchgang betrieben wird.

4. Verfahren nach einem der Ansprüche 1 bis 3,
**dadurch gekennzeichnet,**
**dass** die Hydrierung in einer Vorrichtung durchgeführt wird, die mehr als drei Hydriereinheiten aufweist.

5. Verfahren nach einem der Ansprüche 1 bis 3,
**dadurch gekennzeichnet,**
**dass** die Hydrierung in einer Vorrichtung durchgeführt wird, die drei Hydriereinheiten aufweist.

6. Verfahren nach einem der Ansprüche 1 bis 5,
**dadurch gekennzeichnet,**
**dass** ein Katalysator, der mindestens ein Metall der achten Nebengruppe des Periodensystems der Elemente enthält, verwendet wird.

7. Verfahren nach Anspruch 6,
**dadurch gekennzeichnet,**
**dass** ein Katalysator eingesetzt wird, der Ruthenium enthält.

8. Verfahren nach einem der Ansprüche 1 bis 7,
**dadurch gekennzeichnet,**
**dass** ein Katalysator eingesetzt wird, der einen Titandioxid-Träger aufweist.

9. Verfahren nach einem der Ansprüche 1 bis 8,
**dadurch gekennzeichnet,**
**dass** als aromatische Carbonsäureester Mono-, Di- oder Polycarbonsäureester eingesetzt werden.

10. Verfahren nach einem der Ansprüche 1 bis 9,
**dadurch gekennzeichnet,**
**dass** als aromatische Di- oder Polycarbonsäureester Benzol-, Diphenyl-, Naphthalin-, Diphenyloxid- oder Anthracen-Di- oder Polycarbonsäureester eingesetzt werden.

11. Verfahren nach Anspruch 9,
**dadurch gekennzeichnet,**
**dass** als aromatische Monocarbonsäureester Ester der Benzoesäure, 1-Naphthoesäure oder 2-Naphthoesäure eingesetzt werden.

12. Verfahren nach Anspruch 10,
**dadurch gekennzeichnet,**
**dass** bei Einsatz von Di- oder Polycarbonsäureestern die Partial- und/oder Vollester eingesetzt werden.

13. Verfahren nach Anspruch 1 oder 12
**dadurch gekennzeichnet,**
**dass** die Alkoholkomponenten der eingesetzten aromatischen Carbonsäureester verzweigte oder unverzweigte Alkoxyalkyl-, Cycloalkyl- und/oder Alkylgruppen mit 1 bis 25 Kohlenstoffatomen aufweisen.

14. Verfahren nach zumindest einem der Ansprüche 12 oder 13,
**dadurch gekennzeichnet,**
**dass** die Alkoholkomponenten der eingesetzten aromatischen Di- und/oder Polycarbonsäureester jeweils gleich oder unterschiedlich sind.

15. Verfahren nach zumindest einem der Ansprüche 9 bis 13,
**dadurch gekennzeichnet,**
**dass** als aromatischer Dicarbonsäureester Diisononylphthalat oder Didecylphthalat eingesetzt wird.

16. Verfahren nach Anspruch 11,
**dadurch gekennzeichnet,**
**dass** als aromatischer Monocarbonsäureester Isononylbenzoat oder Decylbenzoat eingesetzt wird.

17. Verfahren nach zumindest einem der Ansprüche 1 bis 16,
**dadurch gekennzeichnet,**
**dass** das Verhältnis der Verweilzeiten in den aufeinanderfolgenden Schlaufenreaktoren von 0,01 bis kleiner 1 beträgt.

## Claims

1. Method for the continuous preparation of alicyclic carboxylic esters by catalytic hydrogenation of the corresponding aromatic carboxylic esters using a hydrogen-containing gas in the presence of solid catalysts disposed in a fixed bed, **characterized in that** the hydrogenation is carried out in at least three series-connected hydrogenation units, **in that** at least the first two hydrogenation units are operated in loop operating mode and **in that** at least two hydrogenation units which are series-connected and operated in loop operating mode are operated with different residence times, the residence time in one of the hydrogenation units operated in loop operating mode being less than in a directly following hydrogenation unit operated in loop operating mode.

2. Method according to Claim 1,
**characterized in that**
all hydrogenation units are operated in loop operating mode.

3. Method according to Claim 1,
**characterized in that**
the last hydrogenation unit is operated in straight through-flow passage.

4. Method according to one of Claims 1 to 3,
**characterized in that**
the hydrogenation is carried out in an apparatus which has more than three hydrogenation units.

5. Method according to one of Claims 1 to 3,
**characterized in that**
the hydrogenation is carried out in an apparatus which has three hydrogenation units.

6. Method according to one of Claims 1 to 5,
**characterized in that**
use is made of a catalyst which comprises at least one metal of the eighth subgroup of the Periodic Table of the Elements.

7. Method according to Claim 6,
**characterized in that**
use is made of a catalyst which comprises ruthenium.

8. Method according to one of Claims 1 to 7,
**characterized in that**
use is made of a catalyst which has a titanium dioxide support.

9. Method according to one of Claims 1 to 8,
**characterized in that**
as aromatic carboxylic esters, use is made of mono-, di- or polycarboxylic esters.

10. Method according to one of Claims 1 to 9,
**characterized in that**
as aromatic di- or polycarboxylic esters, use is made of benzene-, diphenyl-, naphthalene-, diphenyl oxide- or anthracene di- or polycarboxylic esters.

11. Method according to Claim 9,
**characterized in that**
as aromatic monocarboxylic esters, use is made of esters of benzoic acid, 1-naphthoic acid, or 2-naphthoic acid.

12. Method according to Claim 10,
**characterized in that**
when di- or polycarboxylic esters are used, the partial and/or full esters are used.

13. Method according to Claim 1 or 12,
**characterized in that**
the alcohol components of the aromatic carboxylic esters used have branched or unbranched alkoxyalkyl, cycloalkyl and/or alkyl groups having 1 to 25 carbon atoms.

14. Method according to at least one of Claims 12 or 13,
**characterized in that**
the alcohol components of the aromatic di- and/or polycarboxylic esters used are in each case identical or different.

15. Method according to at least one of Claims 9 to 13,
**characterized in that**
as aromatic dicarboxylic ester, use is made of diisononyl phthalate or didecyl phthalate.

16. Method according to Claim 11,
**characterized in that**
as aromatic monocarboxylic ester, use is made of isononyl benzoate or decyl benzoate.

17. Method according to at least one of Claims 1 to 16,
**characterized in that**
the ratio of the residence times in the series-connected loop reactors is from 0.01 to less than 1.

## Revendications

1. Procédé pour la préparation continue d'esters d'acides carboxyliques alicycliques par hydrogénation catalytique des esters d'acides carboxyliques aromatiques correspondants sur des catalyseurs fixes disposés dans un lit fixe avec un gaz contenant de l'hydrogène, **caractérisé en ce que** l'hydrogénation est réalisée dans au moins trois unités d'hydrogénation disposées les unes derrière les autres, **en ce qu'**au moins les deux premières unités d'hydrogénation sont opérées dans un mode en boucle et **en ce qu'**au moins deux unités d'hydrogénation commutées en série et opérées en mode en boucle sont opérées avec des durées de séjour différentes, le temps de séjour dans une des unités d'hydrogénation opérées en mode en boucle étant inférieur à celui dans une unité d'hydrogénation opérée en mode en boucle immédiatement consécutive.

2. Procédé selon la revendication 1, **caractérisé en ce que** toutes les unités d'hydrogénation sont opérées en mode en boucle.

3. Procédé selon la revendication 1, **caractérisé en ce que** la dernière unité d'hydrogénation est opérée en passage droit.

4. Procédé selon l'une quelconque des revendications 1 à 3, **caractérisé en ce que** l'hydrogénation est réalisée dans un dispositif qui présente plus de trois unités d'hydrogénation.

5. Procédé selon l'une quelconque des revendications 1 à 3, **caractérisé en ce que** l'hydrogénation est réalisée dans un dispositif qui présente trois unités d'hydrogénation.

6. Procédé selon l'une quelconque des revendications 1 à 5, **caractérisé en ce qu'**on utilise un catalyseur qui contient au moins un métal du huitième groupe secondaire du système périodique des éléments.

7. Procédé selon la revendication 6, **caractérisé en ce qu'**on utilise un catalyseur, qui contient du ruthénium.

8. Procédé selon l'une quelconque des revendications 1 à 7, **caractérisé en ce qu'**on utilise un catalyseur, qui contient un support en dioxyde de titane.

9. Procédé selon l'une quelconque des revendications 1 à 8, **caractérisé en ce qu'**on utilise comme esters d'acides carboxyliques aromatiques des esters d'acides monocarboxyliques, dicarboxyliques ou polycarboxyliques.

10. Procédé selon l'une quelconque des revendications 1 à 9, **caractérisé en ce qu'**on utilise comme esters d'acides dicarboxyliques ou polycarboxyliques aromatiques des esters dicarboxyliques ou polycarboxyliques de benzène, de diphényle, de naphtalène, de diphényloxyde ou d'anthracène.

11. Procédé selon la revendication 9, **caractérisé en ce qu'**on utilise comme esters d'acides monocarboxyliques aromatiques des esters de l'acide benzoïque, de l'acide 1-naphtoïque ou de l'acide 2-naphtoïque.

12. Procédé selon la revendication 10, **caractérisé en ce qu'**on utilise, lors de l'utilisation d'esters d'acides dicarboxyliques ou polycarboxyliques, des esters partiels et/ou complets.

13. Procédé selon la revendication 1 ou 12, **caractérisé en ce que** les composants alcool des esters d'acides carboxyliques aromatiques utilisés présentent des groupes alcoxyalkyle, cycloalkyle et/ou alkyle ramifiés ou non ramifiés, comprenant 1 à 25 atomes de carbone.

14. Procédé selon au moins l'une quelconque des revendications 12 ou 13, **caractérisé en ce que** les composants alcool des esters d'acides dicarboxyliques et/ou polycarboxyliques aromatiques utilisés sont à chaque fois identiques ou différents.

15. Procédé selon au moins l'une quelconque des revendications 9 à 13, **caractérisé en ce qu'**on utilise comme esters d'acides dicarboxyliques aromatiques du naphtalate de diisononyle ou du phtalate de didécyle.

16. Procédé selon la revendication 11, **caractérisé en ce qu'**on utilise comme esters d'acides monocarboxyliques aromatiques du benzoate d'isononyle ou du benzoate de décyle.

17. Procédé selon au moins l'une quelconque des revendications 1 à 16, **caractérisé en ce que** le rapport des temps de séjour dans les réacteurs à boucle consécutifs est de 0,01 à moins de 1.
